# FASCICULE DE BREVET EUROPEEN

(11) **EP 0 709 061 B1**
(45) Date de publication et mention de la délivrance du brevet: **02.07.2003**
(21) Numéro de dépôt: 95420299.0
(22) Date de dépôt: 27.10.1995
(51) Int. Cl.: A61B 17/15

(54) **Instrumentation ancillaire fémorale pour l'implantation d'une prothèse unicompartimentale du genou**
Femorales Hilfsgerät zur Implantation einer einseitigen Knieprothese
Femoral ancillary instrumentation for implanting a unicompartmental knee prosthesis

(30) Priorité: 27.10.1994 FR 9413095
(43) Date de publication de la demande: 01.05.1996
(73) Titulaire: Merck Biomaterial France, 26000 Valence (FR); Bascoulergue, Gérard, F-62520 Le Touquet (FR); Charret, Philippe, 69270 Fontaine sur Saone (FR); Fayard, Jean-Philippe, F-42170 St. Just St. Rambert (FR); Hulin, Paul Henri, 89000 Auxerre (FR); Peyrot, Jacques, 69160 Tassin la Demi Lune (FR)
(72) Inventeur: Bascoulergue, Gérard, F-62520 Le Touquet (FR); Charret, Philippe, F-69270 Fontaine sur Saone (FR); Dupré-Latour, Laurent, F-42580 L'Etrat (FR); Fayard, Jean-Philippe, F-42170 St Just St Rambert (FR); Hulin, Paul Henri, F-89000 Auxerre (FR); Peyrot, Jacques, F-69160 Tassin la Demi-Lune (FR); Collomb, Jean, F-26800 Portes les Valence (FR)
(74) Mandataire: Ropital-Bonvarlet, Claude

(56) Documents cités:
- EP-A- 0 538 153
- FR-A- 2 664 157
- FR-A- 2 679 766

## Description

La présente invention concerne le domaine de la chirurgie orthopédique et elle vise, plus particulièrement, le matériel mis en oeuvre à titre d'aide opératoire pour la réalisation d'interventions chirurgicales visant à substituer un système articulaire artificiel à une articulation naturelle défaillante.

Dans le domaine technique général ci-dessus, il est connu, depuis longtemps déjà, de proposer des systèmes articulaires artificiels venant se substituer à l'articulation naturelle constituée par la conformation épiphysaire basse ou distale du fémur, par la conformation épiphysaire complémentaire haute ou proximale du tibia et par l'élément fémoro-patellaire.

De telles propositions s'appuient sur la constitution d'un système articulaire artificiel, à partir de deux éléments principaux destinés à être implantés, après résection osseuse, sur la conformation épiphysaire basse du fémur et sur la conformation épiphysaire complémentaire haute du tibia.

Deux types de propositions de substitution peuvent être envisagés selon l'intervention devant être conduite. Il peut s'agir d'un remplacement pluricompartimental simultané, tant pour la conformation épiphysaire du fémur que pour celle du tibia, ou encore d'un remplacement unicompartimental lorsqu'une partie des surfaces congruentes de l'articulation seulement est détériorée.

L'invention concerne spécifiquement le domaine de la chirurgie orthopédique fémorale et le matériel spécifique pour la réalisation de la résection unicompartimentale d'un massif condylien fémoral, interne ou externe, préalablement à l'implantation d'une prothèse unicompartimentale.

Dans une telle situation, l'intervention consiste alors à adapter après résection des parties congruentes unicompartimentales et complémentaires de l'articulation concernée, une prothèse unicompartimentale du tibia et une prothèse unicompartimentale du massif condylien concerné du fémur.

Une intervention, du type ci-dessus, concerne obligatoirement une masse osseuse qui est de plus faible importance que celle intéressée par l'implantation d'une prothèse pluricompartimentale. Il est donc particulièrement important de pouvoir exécuter la ou les coupes de résection osseuse avec une précision certaine pour permettre une implantation d'une prothèse unicompartimentale dont la faible épaisseur et la largeur réduite exigent une implantation précise respectant les caractéristiques anatomiques de l'articulation et, notamment, celles régissant le bon fonctionnement de l'articulation semi-artificielle et semi-naturelle qui en résultera.

Il convient, en effet, de considérer que dans une telle situation, l'obligation à respecter est de pouvoir implanter une prothèse unicompartimentale, de manière qu'elle vienne compléter le compartiment naturel subsistant dans la fonctionnalité de l'articulation et qu'elle puisse présenter, dans le temps et malgré les sollicitations qu'elle subit, une implantation osseuse ferme et résistante, garantissant une bonne tenue dans le temps.

Si des prothèses unicompartimentales, du genre ci-dessus, ont déjà été proposées dans la technique orthopédique, en revanche, il peut être considéré que l'instrumentation ancillaire existante ne permet pas de respecter justement la conduite avec précision des différentes coupes de résection osseuse qu'il convient d'exécuter pour assurer une mise en place d'une telle prothèse répondant aux objectifs ci-dessus.

La demande EP 0 538 153 décrit, par exemple, un ancillaire fémoral pour l'exécution d'une résection bicompartimentale du fémur. Cet ancillaire comprend, notamment :
- une colonne pourvue, à une extrémité, de deux pattes interstitielles destinées à être engagées dans les intervalles intercondyliens,
- une pièce d'appui empilable sur la colonne et comportant, dans la même direction que la patte, deux organes de butée définissant un plan de contact simultané avec les parties distales du fémur et, à l'opposé de ces organes, un fourreau tubulaire traversant la pièce,
- un guide de coupe réglable sur une règle montée sur la colonne à l'opposé de la patte par rapport à la pièce d'appui et s'étendant latéralement par rapport à la colonne et parallèlement au plan d'appui, pour permettre l'exécution, selon une orientation parallèle audit plan, de la coupe distale (**CD**) des compartiments condyliens, le guide de coupe comportant des perçages permettant l'engagement de broches d'immobilisation dans les deux massifs du fémur,
- et une tige de visée centro-médullaire engageable dans le fourreau et dans le canal médullaire du fémur à partir de l'échancrure intercondylienne.

Selon cette demande EP 0 538 153, l'immobilisation finale du guide de coupe avant résection est effectuée au moyen de broches engagées dans les deux massifs du fémur. Or, pour la mise en place d'une prothèse unicompartimentale, il est nécessaire de ne pas altérer le compartiment sain de l'articulation. De plus, pour offrir à une telle prothèse des conditions d'implantation les meilleures possibles, il convient de rechercher, autant que faire se peut, des points d'ancrage n'affectant pas la partie du massif osseux devant être réséquée. Enfin, l'ancillaire doit comporter, en outre, des moyens d'immobilisation fiables qui garantissent l'invariabilité de sa position par rapport au fémur pendant toute l'exécution de la résection.

Il semble que l'art antérieur ne propose pas d'instrumentation ancillaire répondant à l'ensemble de ces exigences, pourtant complémentaires.

L'objet de l'invention est justement de combler cette lacune en proposant une instrumentation ancillaire fémorale qui soit propre à l'implantation d'une prothèse unicompartimentale du fémur dont la conception est justement prévue aussi pour permettre une bonne implantation sans conduire à une résection compartimentale osseuse importante.

L'objet de l'invention est de proposer une instrumentation ancillaire fémorale, simple de conception et d'utilisation et dont les moyens techniques permettent de déterminer avec précision l'orientation, l'alignement et la position des moyens d'aide à l'implantation permettant de réaliser des résections osseuses selon des plans relatifs, favorables à la bonne implantation d'une prothèse unicompartimentale du fémur.

L'objet de l'invention vise une instrumentation ancillaire fémorale à même de favoriser le travail préparatoire du chirurgien, en lui fournissant des axes et plans de référence et d'appui, à position, orientation ou sens, éventuellement réglables, définissant un domaine référentiel précis à partir duquel une implantation de prothèse fémorale unicompartimentale peut être réalisée avec une sécurité maximale.

Pour atteindre les objectifs ci-dessus, l'instrumentation ancillaire fémorale est caractérisée en ce qu'elle comprend :
- un guide de coupe distale comportant une barrette latérale pourvue de trous pour l'engagement de broches,
- et un bloc de coupes postérieure et intermédiaire adaptable par broches sur la coupe distale.

Diverses autres caractéristiques ressortent de la description faite ci-dessous en référence aux dessins annexés qui montrent, à titre d'exemples non limitatifs, des formes de réalisation de l'objet de l'invention.

La **fig. 1** est une vue de face partielle d'un genou gauche en position fléchie et montrant l'implantation d'une prothèse unicompartimentale sur le massif condylien interne.

La **fig. 2** est une élévation, partie en coupe, prise selon la ligne II-II de la **fig. 1.**

La **fig. 3** est une élévation analogue à la **fig. 2,** mais montrant une partie de l'instrumentation ancillaire conforme à l'invention.

La **fig. 4** est une vue de face prise sensiblement selon la ligne IV-IV de la **fig. 3.**

La **fig. 5** est une vue de dessus prise selon la ligne V-V de la **fig. 3.**

La **fig. 6** est une élévation analogue à la **fig. 3,** mais montrant une variante de réalisation.

La **fig. 7** est une perspective montrant, à plus grande échelle, une autre partie de l'instrumentation ancillaire conforme à l'invention.

La **fig. 8** est une perspective vue selon une autre orientation de la partie d'instrumentation selon la **fig. 7,** pourvue de son équipement d'implantation.

La **fig. 9** est une vue analogue à la **fig. 3,** mais montrant la mise en oeuvre de la partie d'instrumentation selon les **fig. 7** et **8.**

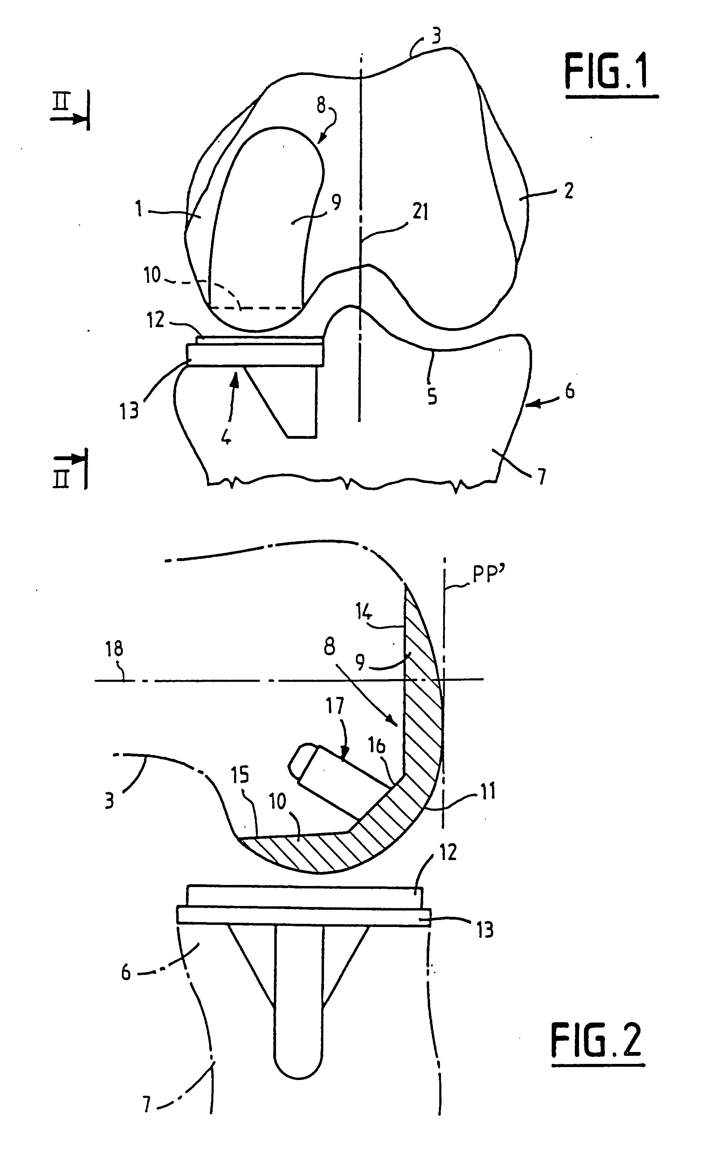

Les **fig. 1** et **2** montrent, de façon schématique, une articulation de genou gauche faisant intervenir la coopération de surfaces congruentes, tels que les condyles interne **1** et externe **2** d'un fémur **3** avec les glènes **4** et **5** du plateau **6** d'un tibia **7.**

Dans une articulation naturelle, du type ci-dessus, l'adaptation d'une prothèse unicompartimentale du fémur consiste à implanter, par exemple, en substitution du condyle interne **1**, une prothèse unicompartimentale **8** réalisée sous la forme d'un élément prothétique sensiblement en équerre, comportant une branche **9** dite de condyle distal et une branche **10** dite de condyle postérieur. Les branches **9** et **10** définissent ensemble une surface extérieure **11** courbe, qui est destinée à coopérer avec la surface supérieure d'un patin **12** d'une prothèse unicompartimentale de tibia **13,** uniquement schématisée à titre d'exemple aux **fig. 1** et **2** et implantée en remplacement de la glène **4.** Les branches **9** et **10** sont définies, par ailleurs, par des surfaces planes arrières **14** et **15** qui, de préférence, sont raccordées par un pan incliné **16** à partir duquel ou au voisinage duquel un moyen d'insertion osseuse **17** est prévu, par exemple, sous la forme d'un ou de plusieurs tétons.

L'implantation d'une prothèse unicompartimentale, du type ci-dessus, implique de pouvoir réaliser une résection osseuse du massif condylien **1** de manière complémentaire aux face d'adaptation **14, 15** et **16,** tout en respectant la relation d'orientation avec l'axe anatomique **18** du fémur et avec le plan articulaire naturel défini par l'alignement des condyles **1** et **2** considérés selon un plan distal extrême, tel que le plan **P-P'.**

L'instrumentation ancillaire fémorale selon l'invention est justement conçue pour fournir une aide opératoire propre à l'exécution des résections osseuses, nécessaires et complémentaires à l'implantation de la prothèse unicompartimentale **8.**

Ce qui vaut pour le cas d'une prothèse **8** interne de genou gauche vaut également pour une prothèse interne de genou droit, qui peut être considérée comme symétrique par rapport à un plan sagittal et, toute adaptation nécessaire étant prévue, également pour une prothèse unicompartimentale externe gauche ou externe droite pour les mêmes raisons. L'instrumentation ancillaire fémorale est justement prévue pour répondre à un tel besoin global, en faisant intervenir des moyens technique principaux valant pour toutes les applications et en ne prévoyant, en fonction des compartiments articulaires visés, que des adaptations partielles de pièces élémentaires, sans que ces adaptations aient une incidence sur la combinaison globale des moyens techniques mis en oeuvre.

L'instrumentation ancillaire fémorale comprend tout d'abord un moyen **20** de visée centro-médullaire qui est engageable dans le canal médullaire du fémur selon l'axe anatomique **18,** à partir de l'échancrure intercondylienne **21** (**fig. 1**). Un tel canal médullaire fait généralement l'objet d'un percement qui est pratiqué de manière connue, pour permettre l'insertion du moyen **20.**

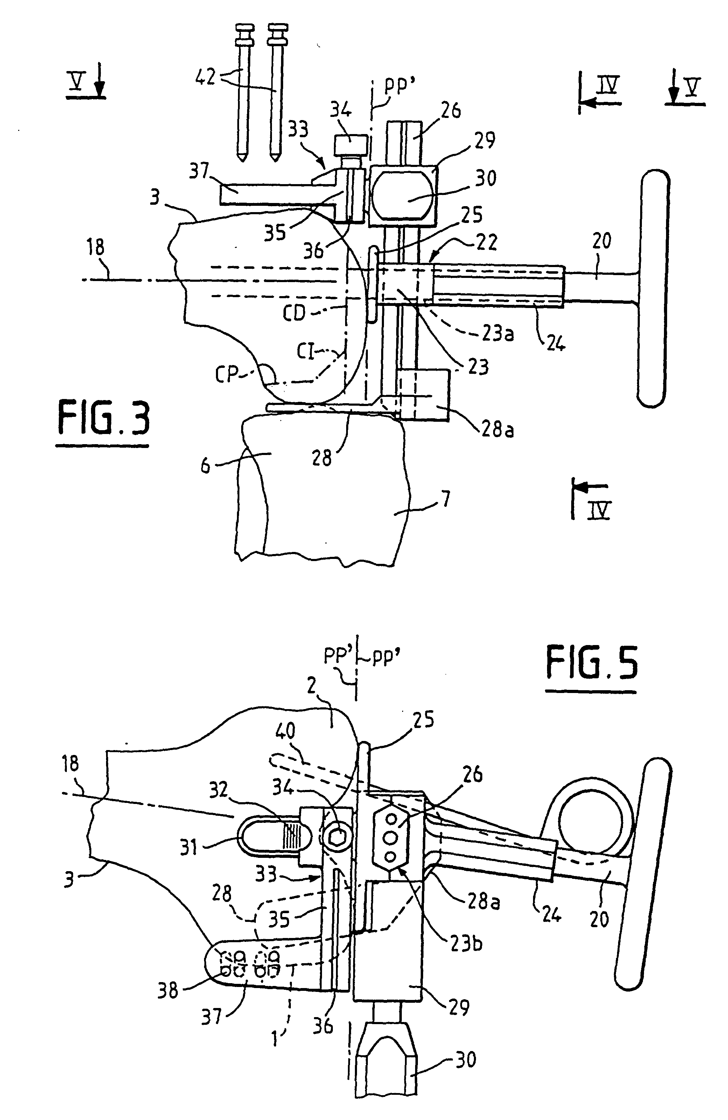

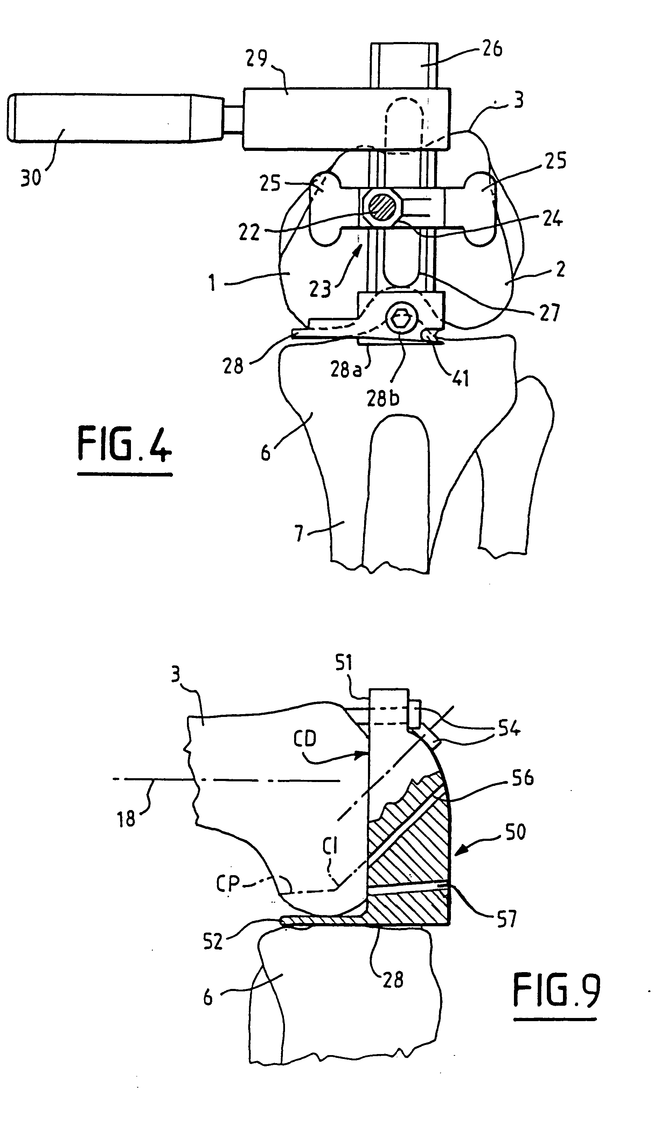

Selon une disposition de l'invention, le moyen **20** est constitué par une tige-guide propre à assurer le guidage d'une pièce **22** dite d'appui en raison de la fonction qu'elle doit assumer. La pièce d'appui **22** comporte un corps central **23** traversé par un trou **23a** et prolongé d'un côté par un fourreau **24** coïncidant avec le trou **23a** et susceptible d'être enfilé sur la tige **20.** Le corps central **23** comporte à l'opposé du fourreau **24,** deux organes de butée **25** qui définissent un plan **p-p'** de contact simultané avec les parties distales des condyles **1** et **2** du fémur, tel que cela ressort des **fig. 3** à **5.** Les organes de butée **25** dont destinés à matérialiser le plan **p-p'** pour définir une position de référence, lors de la mise en place de l'instrumentation, comme cela ressort de ce qui suit.

L'instrumentation comprend, par ailleurs, une colonne **26** qui est engagée libre dans un logement **23b** complémentaire présenté par le corps central **23** selon une direction orthogonale à celle du trou **23a.** La colonne **26,** une fois montée dans le logement **23b** s'étend, en considération de la **fig. 3,** selon une direction orthogonale à l'axe du fourreau **24.** La colonne **26** et le logement **23b** sont de section polygonale complémentaire, interdisant toute rotation relative. La colonne **26** présente, par ailleurs, sur une partie médiane de sa longueur, une lumière **27** axiale prévue pour le passage de la tige **20** et pour autoriser son déplacement par rapport au corps central **23** selon une direction qui est parallèle au plan **p-p'.**

La colonne **26** est munie, à l'une de ses extrémités, d'une patte interstitielle **28** amovible, d'épaisseur généralement constante, qui s'étend parallèlement à l'axe du fourreau **24** et perpendiculairement à la colonne **26** dans la direction des organes de butée **25.** La patte **28** est, par exemple, montée sur la colonne **26** par un talon **28a** pourvu d'un organe de serrage, tel que **28b.** Le talon **28a** présente un emboîtement complémentaire à la section polygonale de la colonne interdisant toute rotation relative.

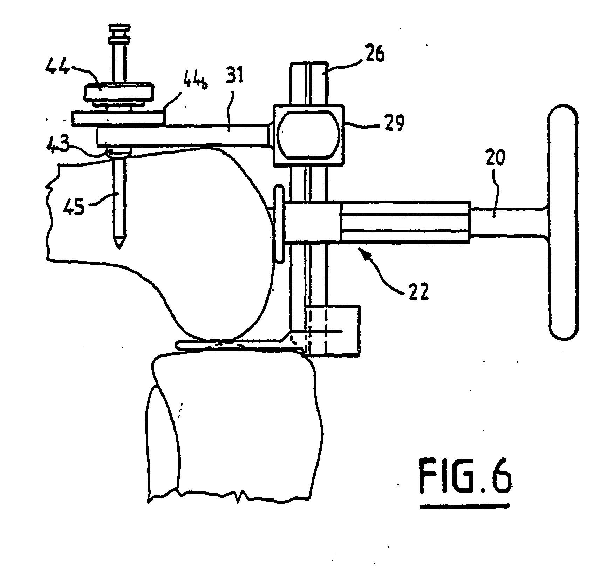

La **fig. 5** permet de constater que, selon l'invention, le talon **28a** est conçu pour que la patte **28** s'étende latéralement, de manière à pouvoir être placée en relation et à l'aplomb d'un massif condylien, tel que le massif **1** dans l'exemple illustré, comme cela apparaît dans ce qui suit.

La partie de la colonne **26** s'étendant au delà du corps central **23** par rapport à la patte **28** porte un coulisseau porte-guide de coupe **29,** à position axiale réglable par l'intermédiaire, par exemple, d'une manette **30** ou analogue. Le coulisseau **29** est prolongé, dans le même sens que la patte **28,** par une règle **31** (**fig. 5**) s'étendant perpendiculairement à la colonne **26.** La règle **31** est prévue pour le montage, en position réglable, notamment par l'intermédiaire de graduations **32,** d'un guide de coupe distale **33** pouvant être immobilisé en position appropriée par l'intermédiaire d'un organe de blocage **34.** Le guide de coupe distale **33** comprend un corps **35** s'étendant de façon latéralement déportée dans le même sens que la patte **28** et parallèlement à cette dernière. Le corps **35** délimite une fente guide de coupe **36** dont le plan, tel que cela apparaît aux **fig. 3** et **5,** est parallèle à celui de la colonne **26** et au plan **p-p',** en étant situé toutefois au-delà de ce dernier par rapport à la colonne **26.** Le corps **35** est prolongé par une barrette latérale **37** pourvue de trous **38** dont la fonction apparaît dans ce qui suit.

La mise en oeuvre de la partie d'instrumentation ancillaire fémorale décrite ci-dessus est assurée de la façon suivante pour l'exécution de la résection osseuse distale du condyle **1** interne d'un genou gauche.

Après le percement du canal centro-médullaire selon l'axe anatomique **18,** la colonne **26** est présentée face à l'épiphyse du fémur avec flexion du genou selon la **fig. 3,** pour assurer l'engagement de la patte **28** entre le condyle fémoral **1** et la partie complémentaire appareillée ou non du tibia **7.** La pièce **22** est enfilée librement sur la colonne **26** pour présenter les organes de butée **25** en direction des condyles fémoraux **1** et **2.** La pièce **22** est placée pour mettre en coïncidence le trou **23a** avec le canal médullaire percé, de manière à permettre l'engagement de la tige de visée **20.**

La colonne **26** est alors poussée pour engager plus profondément la patte **28** et amener le plan **p-p'** en contact avec les parties distales des condyles fémoraux **1** et **2,** le plan **p-p'** étant alors confondu avec l'alignement anatomique **P-P'.**

Le coulisseau **29** est ensuite monté sur la colonne **26,** puis le guide **33** est enfilé sur la règle **31.** Le coulisseau **29** est réglé pour amener le guide en contact avec la face antérieure du massif condylien **1,** puis le guide est réglé sur la règle **31,** de manière à déterminer par le plan de la fente **36** le plan de coupe distale **CD.**

Dans cet état, l'instrumentation est préférablement immobilisée par l'engagement de broches transosseuses **42,** dans les trous **38** de la barrette **37** dont le déport latéral, la longueur ainsi que l'orientation des trous, permettent une pénétration des broches dans une zone du massif condylien **1** qui n'est pas concernée par la résection osseuse devant être pratiquée.

Pour disposer d'un équilibre d'orientation, il peut être envisagé d'assurer l'insertion interstitielle d'une cale d'épaisseur **40** dans l'intervalle intercondylien entre le condyle **2** et la glène **5.** La cale d'épaisseur **40** peut être constituée par une tige amovible qui est, par exemple, insérable dans un logement **41** ménagé par le talon d'adaptation de la patte **28.**

Une lame de scie appropriée peut alors être engagée par la fente de coupe **36** selon un plan parallèle aux plans confondus **P-P'** et **p-p',** de manière à réaliser la coupe distale **CD** de la partie du condyle **1,** sur une profondeur ou une épaisseur juste nécessaire pour recevoir l'épaisseur de la branche **9** de la prothèse unicompartimentale **8** décrite précédemment.

Etant donné que le plan de la fente **36** est réglé avec précision par l'organe **34** parallèlement aux plans **P-P'** et **p-p',** une coupe distale **CD** intervient obligatoirement sur une profondeur osseuse précise, déterminée et selon une direction qui correspond exactement à un plan parallèle au plan anatomique **P-P'.**

Pour que le plan de la fente **36** soit exactement orienté parallèlement aux plans confondus **P-P'** et **p-p',** il peut être avantageux de prolonger la règle **31,** comme montré par la **fig. 6.** Dans cette variante, la partie terminale de la règle **31** porte un doigt **43** réglable par un bouton **44.** La règle **31** et la patte **28,** éventuellement complétée par la cale **40,** forment alors un étau ou une pince d'immobilisation du fémur. Lorsque le guide **33** est placé, comme dit précédemment, en butée contre la face antérieure du massif **1,** le doigt **43** est réglé pour venir en butée contre le fémur et le serrer, de manière à empêcher tout basculement de l'instrumentation ancillaire et toute flexion de la règle **31,** notamment lors de la mise en place des broches **42.** Il peut être envisagé aussi d'associer le doigt **43** à une broche traversante **45** contribuant, après mise en butée du doigt, à l'immobilisation de la règle **31.** Enfin, le doigt réglable peut comporter un contre-bouton **44b** permettant d'éviter tout desserrage accidentel.

Il doit être remarqué que l'exécution de cette résection osseuse selon le plan **CD** n'intéresse que la partie distale du condyle **1** et qu'elle peut être conduite en toute sécurité, sans risque de pénétration intempestive dans la glène **4** complémentaire du tibia **7** ou dans la prothèse **13,** étant donné que la patte interstitielle **28** constitue une butée positive pour l'extrémité de la scie.

Lorsque la coupe distale **CD** a été effectuée, la partie de l'instrumentation ancillaire fémorale décrite ci-dessus est ôtée en procédant de façon inverse à ce qui est décrit précédemment, de manière à libérer l'articulation du genou et à dégager le plan de résection osseuse résultant de la coupe distale du condyle **1**.

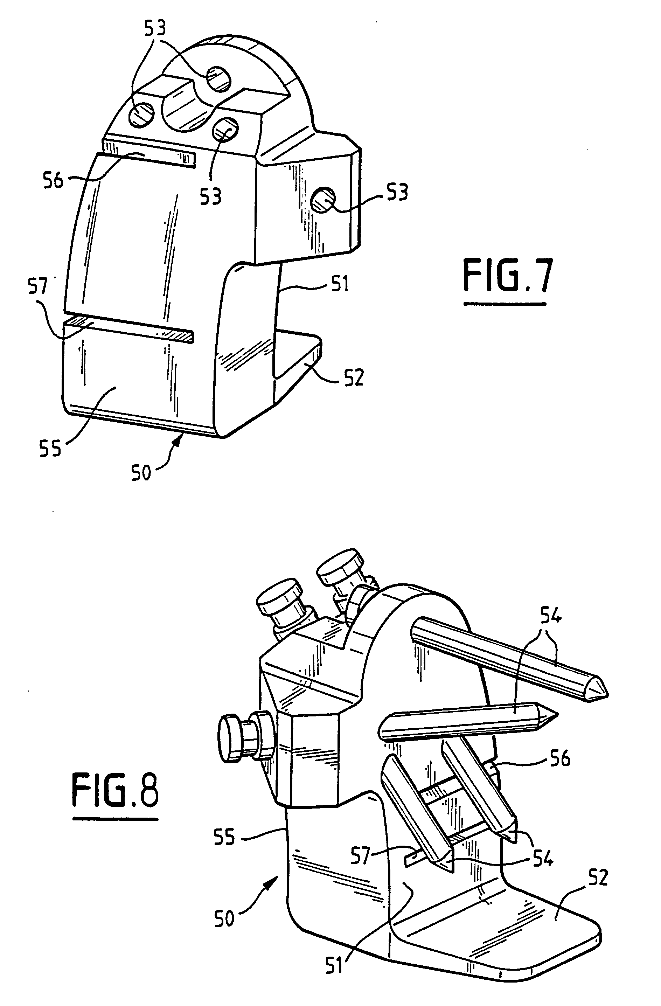

La préparation nécessaire à l'adaptation et l'insertion de la prothèse unicompartimentale **8** fait alors intervenir une deuxième partie d'instrumentation ancillaire fémorale, telle qu'illustrée par les **fig. 7** et **8.** Cette seconde partie d'instrumentation ancillaire fémorale comprend un bloc **50** dit de coupes intermédiaire et postérieure. Le bloc **50** est réalisé sous la forme d'une pièce ou d'un corps massif possédant une face plane de référence **51** à partir de laquelle s'étend une patte interstitielle **52** ménagée dans le prolongement de l'extrémité correspondante du bloc **50.**

La partie du bloc opposée à la patte **52** présente des alésages cylindriques traversants **53,** par exemple au nombre de quatre, qui sont pratiqués suivant des directions différentes pour permettre l'engagement de broches **54** d'insertion osseuse temporaire. Le choix des directions différentes est effectué pour que l'engagement des broches **54** établisse une sorte de triangulation interne favorable à une immobilisation ferme et résistante du bloc **50,** lors de sa mise en place comme exposé ci-après.

Le bloc **50** présente, par ailleurs, deux fentes-guides de coupe qui sont pratiquées à partir de la face **55** opposée à la face plane **51,** en débouchant, de préférence mais non obligatoirement, sur l'une des faces transversales. La première fente-guide de coupe **56** est pratiquée selon une inclinaison orientée vers la patte **52** et pour déboucher sur la face plane **51**. La seconde fente-guide de coupe **57** est pratiquée de façon sensiblement parallèle à la patte **52** pour s'ouvrir sur la face **51** entre le plan de cette dernière et la sortie de la fente **56.** De manière préférée, la seconde fente **57** présente une inclinaison de l'ordre de 4° en direction de la patte **52.**

Le bloc **50** décrit ci-dessus est adapté sur la partie distale du fémur **3,** tel que cela est illustré par la **fig. 8,** de manière que la patte interstitielle **52** soit engagée dans l'espace intercondylien entre le condyle **1** et la cavité glénoïde **4** ou la prothèse **13** et de telle sorte que la face de référence **51** vienne prendre appui avec la face de résection osseuse **CD** correspondant à la coupe distale précédemment exécutée.

Dans cette position, l'immobilisation du bloc **50** est assurée par l'engagement des broches **54** dans les différents alésages **53.**

Par l'intermédiaire d'une scie engagée à partir de la face **55** du bloc **50,** il peut alors être procédé à l'exécution, par l'intermédiaire de la fente **56,** d'une résection intermédiaire visant à ménager un chanfrein dans le condyle **1** à partir de la coupe distale **CD,** de telle sorte que soit produite, comme illustré en traits mixtes à la **fig. 3,** une coupe intermédiaire **CI,** complémentaire au pan incliné **16** de la prothèse **8.**

De la même manière, une coupe postérieure **CP** est exécutée par une même lame de scie engagée dans la fente-guide de coupe **57** pour réséquer la partie postérieure du condyle **1** sur une épaisseur prédéterminée par la conformation du bloc **50** et correspondant à celle de la branche **10** de la prothèse **8.**

Le bloc **50** peut ensuite être extrait par dégagement des broches **54** pour mettre à nu les trois plans de résection osseuse, **CD, CI**, **CP,** exécutés sur le condyle **1** pour former la contrepartie d'adaptation et de réception des faces arrières **14, 15** et **16** de la prothèse **8.**

Il convient de noter que dans cette phase préparatoire, seules les faces d'appui et de réception **CD, CI** et **CP** ont été exécutées, de sorte qu'il est possible de faire intervenir une phase d'adaptation intermédiaire consistant à placer, sur ces faces de résection osseuse, un gabarit de pose correspondant à la forme exacte de la prothèse **8** retenue et devant être implantée.

Le cas échéant, il est possible de procéder à toute réadaptation de surface complémentaire au moyen d'une râpe ou analogue, pour parfaire la complémentarité des faces **CD, CI, CP** et **14, 16,** et **15** avant de procéder au percement dans le massif condylien du ou des trous borgnes destinés à recevoir les moyens d'insertion, tels que **17.**

Ainsi que cela ressort des moyens décrits et de leur mise en service, l'instrumentation ancillaire fémorale, conforme à l'invention, permet de faire intervenir des plans de référence correspondant exactement aux caractéristiques anatomiques de l'articulation concernée, pour exécuter des résections osseuses d'épaisseur juste suffisante et complémentaires à l'implantation de la prothèse unicompartimentale, telle que **8.**

De même, l'exécution de ces plans offre une possibilité de correction ultérieure par contrôle d'un gabarit de pose à partir duquel seulement, le ou les percements d'insertion sont exécutés avec la sécurité maximale.

Les moyens mis en oeuvre permettent de protéger l'environnement de l'articulation contre les accidents de sciage susceptibles d'intervenir et, notamment, de préserver l'intégrité du plateau tibial **4** ou de la prothèse **13** par la présence de la patte interstitielle **28** et de la patte **52** laquelle assume la même fonction que la patte **28** pour la coupe intermédiaire selon le plan de coupe **CI.**

Les moyens selon l'invention permettent une adaptation d'application, étant donné qu'il suffit de monter sur la colonne **26,** soit la patte **28** et le guide **33** dans une position symétrique et latéralement déportée à l'opposé de ce qui est représenté dans le cas de la résection du condyle **2,** soit d'une patte **28** et d'un guide de coupe **33** spécialement conformés pour le condyle **2.**

Ce qui vaut pour les condyles **1** et **2** d'un fémur gauche, vaut également pour ceux interne et externe d'un genou droit.

Il en est de même pour ce qui concerne la partie de l'instrumentation constituée par le bloc **50** qui est réalisé de façon symétrique et complémentaire pour l'exécution des résections osseuses **CI** et **CP** du condyle **2** ou des condyles interne et externe d'un genou droit.

L'invention n'est pas limitée aux exemples décrits et représentés car diverses modifications peuvent y être apportées sans sortir de son cadre.

## Revendications

1. Instrumentation ancillaire fémorale pour l'implantation d'une prothèse unicompartimentale du genou, comprenant :
- une colonne (**26**) pourvue à une extrémité d'une patte interstitielle **(28)** déportée latéralement et destinée à être engagée dans l'intervalle intercondylien ménagé par le condyle devant être adapté et la contrepartie tibiale,
- une pièce d'appui **(22)** enfilable sur la colonne et comportant dans la même direction que la patte **(28),** deux organes de butée **(25)** définissant un plan **(p-p')** de contact simultané avec les parties distales **(1, 2)** du fémur et, à l'opposé de ces organes, un fourreau tubulaire **(24)** traversant la pièce d'appui **(22)**,
- un guide de coupe distale **(33)** monté réglable sur une règle **(31)** s'étendant perpendiculairement au plan de contact **(p-p')** de la pièce d'appui **(22**), à partir d'un coulisseau **(29)** réglable sur la colonne, ledit guide de coupe distale **(33)** délimitant une fente de coupe **(36)** dont le plan est parallèle au plan **(p-p')** et pouvant être réglé à distance de ce dernier,
- une tige de visée centro-médullaire **(20)** engageable dans le fourreau tubulaire **(24)** et dans le canal médullaire du fémur **(3)** à partir de l'échancrure intercondylienne,
**caractérisée**
- **en ce que** le guide de coupe **(33)** distale comporte une barrette latérale **(37)** pourvue de trous **(38)** pour l'engagement de broches **(42),**
- et **en ce qu'**elle comprend un bloc **(50)** de coupes postérieure et intermédiaire adaptable par broches sur la coupe distale.

2. Instrumentation ancillaire fémorale selon la revendication 1, **caractérisée :**
- **en ce que** la patte interstitielle **(28)** montée sur la colonne **(26)** par l'intermédiaire d'un talon d'adaptation **(28a)** interdisant toute rotation relative de la patte par rapport à la colonne,
- et **en ce qu'**elle comprend une cale d'épaisseur **(40)** engageable dans un logement **(41)** du talon **(28a)** et destinée à être insérée dans l'intervalle intercondylien du compartiment non concerné par la résection.

3. Instrumentation ancillaire fémorale selon la revendication 1 ou 2, **caractérisée en ce qu'**elle comprend une règle (**31**) pourvue en bout d'un doigt de butée réglable (**43**).

4. Instrumentation ancillaire fémorale selon la revendication 3, **caractérisée en ce que** le doigt (**43**) est réglable par l'intermédiaire d'un bouton (**44**) exerçant une pression sur le fémur et constituant, entre la règle (**31**) et la patte (**28**) une pince d'immobilisation dudit fémur.

5. Instrumentation ancillaire fémorale selon la revendications 3 ou 4, **caractérisée en ce que** le doigt de butée (**43**) est bloqué en position de serrage par un contre-bouton (**44b**).

6. Instrumentation ancillaire fémorale selon la revendications 3 ou 4, **caractérisée en ce que** le doigt de butée (**43**) comporte un perçage axial pour l'engagement d'une broche traversante (**45**).

7. Instrumentation ancillaire fémorale selon la revendication **1, caractérisée en ce que** le bloc **(50)** de coupes intermédiaire et postérieure présente :
- une face plane **(51)** de butée contre la surface de coupe distale **(CD)** du massif condylien,
- une patte interstitielle **(52)** s'étendant depuis la base du bloc à partir de la face plane de butée,
- et deux fentes-guides de coupe **(56, 57**) traversant le bloc depuis la face opposée à la face plane pour s'ouvrir sur cette dernière, la première (**56**) desdites fentes étant inclinée vers la patte à partir de la face opposée à la face plane, tandis que la seconde (**57**) est sensiblement parallèle à la patte et se trouve ménagée entre cette dernière et la fente inclinée.

8. Instrumentation ancillaire fémorale selon la revendication 7, **caractérisée en ce que** la seconde fente (**57**) présente une inclinaison de l'ordre de 4° par rapport à la patte (**52**) et en direction de cette dernière.

9. Instrumentation ancillaire fémorale selon l'une des revendications 1, 7 ou 8, **caractérisée en ce que** la partie du bloc, située à l'opposé de la patte **(52),** possède des alésages traversants **(53)** établis dans plusieurs directions différentes pour l'engagement d'autant de broches **(54)** de fixation temporaire transosseuse.

## Patentansprüche

1. Femorales Hilfsinstrument zur Implantation einer unikondylären Kniegelenkprothese, umfassend:
- eine Säule (26), die an einem Ende mit einer interstitiellen Lasche (28) versehen ist, die seitlich versetzt und dazu bestimmt ist, in den interkondylären Zwischenraum eingeführt zu werden, der durch die anzusetzende Kondyle und das tibiale Gegenstück gebildet ist,
- ein Auflageteil (22), das auf die Säule aufschiebbar ist und das in der gleichen Richtung wie der Arm (28) zwei Anschlagorgane (25) aufweist, die eine Ebene (p-p') bilden, die die distalen Teilen (1, 2) des Femurs gleichzeitig berührt, sowie in entgegengesetzter Richtung zur diesen Organen eine röhrenförmige Hülse (24),die das Auflageteil (22) durchquert,
- eine distale Schnittführung (33), die an einem Maßstab verstellbar angebracht ist, das lotrecht zur Berührungsebene (p-p') des Auflageteils (22) verläuft, ausgehend von einem an der Säule verstellbaren Schieber (29), wobei die distale Schnittführung (33) einen Schnittspalt (36) begrenzt, dessen Ebene parallel zur Ebene (p-p') verläuft und von dieser beabstandet verstellt werden kann,
- eine Stange zur zentralmedullären Visur (20), die in die röhrenförmige Hülse (24) und in die Markhöhle des Femurs (3) ausgehend von dem interkondylären bogenförmigen Ausschnitt einführbar ist,
**dadurch gekennzeichnet, dass**
- die distale Schnittführung (33) einen seitlichen Steg (37) umfasst, der mit Löchern zum Einführen von Stiften (42) versehen ist,
- und dass es eine Einheit (50) hinterer und dazwischenliegender Schnitte umfasst, die durch Stifte an den distalen Schnitt ansetzbar ist.

2. Femorales Hilfsinstrument nach Anspruch ,1
**dadurch gekennzeichnet, dass**
- die interstitielle Lasche (28), die an der Säule (26) mittels einer Befestigungsansatzes (28a) angebracht ist, der jede relative Drehung des Arms bezüglich der Säule verhindert,
- und dass es einen Unterlegkeil (40) umfasst, der in eine Aufnahme (41) des Ansatzes einführbar und dazu bestimmt ist, in den interkondylären Zwischenraum der von der Resektion nicht betroffenen Kammer eingeführt zu werden.

3. Femorales Hilfsinstrument nach Anspruch 1 oder 2,
**dadurch gekennzeichnet, dass** es einen Maßstab (31) umfasst, der am Ende mit einem verstellbaren Anschlagzapfen (43) versehen ist.

4. Femorales Hilfsinstrument nach Anspruch 3,
**dadurch gekennzeichnet, dass** der Zapfen (43) mittels eines Knopfs (44) verstellbar ist, der einen Druck auf den Femur ausübt und zwischen dem Maßstab (31) und der Lasche (28) eine Klemme zum Fixieren des Femurs bildet.

5. Femorales Hilfsinstrument nach Anspruch 3 oder 4,
**dadurch gekennzeichnet, dass** der Anschlagzapfen (43) in der Klemmstellung von einem Gegenknopf blockiert wird.

6. Femorales Hilfsinstrument nach Anspruch 3 oder 4,
**dadurch gekennzeichnet, dass** der Anschlagzapfen (43) eine axiale Bohrung zum Einführen eines durchgehenden Stifts aufweist.

7. Femorales Hilfsinstrument nach Anspruch 1,
**dadurch gekennzeichnet, dass** die Einheit (50) aus dazwischenliegenden und hinteren Schnitte
- eine ebene Seite (51) zum Anschlag gegen die Fläche des distalen Schnitts (CD) des Gelenkkörpers aufweist,
- eine interstitielle Lasche (52) aufweist, die sich von der Basis der Einheit bis zur ebenen Anschlagseite erstreckt,
- und zwei Schnittführungsspalten (56, 57) aufweist, die die Einheit von der Seite aus durchqueren, die der ebenen Seite gegenüber liegt und zur dieser weist, wobei der erste (56) der Spalten von der der ebenen Seite gegenüber liegenden Seite aus in Richtung zu der Lasche geneigt ist, wogegen der zweite Spalt (57) im Wesentlichen parallel zu der Lasche verläuft und zwischen diesem und dem geneigten Spalt gebildet ist.

8. Femorales Hilfsinstrument nach Anspruch 7,
**dadurch gekennzeichnet, dass** der zweite Spalt (57) in Bezug zu der Lasche (52) und in Richtung zu dieser eine Neigung um 4° aufweist.

9. Femorales Hilfsinstrument nach einem der Ansprüche 1, 7 oder 8
**dadurch gekennzeichnet, dass** der Teil der Einheit, die der Lasche (52) gegenüber liegt, durchgehende Bohrungen (53) besitzt, die in mehreren unterschiedlichen Richtungen zum Einführen ebenso vieler Stifte (54) zur vorübergehenden Knochenbefestigung gebildet sind.

## Claims

1. Ancillary femoral instrumentation for implantation of a unicompartmental knee including:
- column (26) equipped at one end with an interstitial tab (28) offset laterally and designed for positioning in the intercondylar notch consisting of modified condyle and corresponding tibial area
- threaded support part (22) attached to the column including two stopper mechanisms (25) facing the same direction as the tab (28) and defining a simultaneous contact plane p-p' with the distal femoral locations (1, 2) and, at the other end, a tubular sleeve (24) lying across the support part (22)
- adjustable distal resection guide (33) mounted on a bar (31) extending perpendicular to contact plane p-p' of the support part (23) from a slide-action adjustment (29) on the column, the distal resection guide (33) marking a guide slot (36) on a plane parallel to plane p-p' and being remotely adjustable
- centromedullary aimer rod (20) configured for entry into the tubular sleeve (24) in the femoral medullary canal (3) from the intercondylar fossa including:
- lateral strip (37) on the distal resection guide (33) with holes (38) to accept the pins (42)
- mount (50) with posterior and intermediate guides fitted for pins on the distal section.

2. Ancillary femoral instrumentation as described in claim 1 and also featuring:
- an interstitial tab (28) mounted on the column (26) using a fitted end (28a) preventing rotation of the tab in relation to the column
- spacer (40) configured to go into a recess (41) in the fitted end (28a) and the intercondylar notch of the compartment not requiring resection.

3. Ancillary femoral instrumentation as described in claim 1 or 2 and also featuring a strip (31) configured at one end with an adjustable locking nail (43).

4. Ancillary femoral instrumentation as described in claim 3 and also featuring a nail (43) adjusted with a button (44) to place pressure on the femur and act as a locking clamp on the femur between the bar (31) and tab (28).

5. Ancillary femoral instrumentation as described in claim 3 or 4 and also featuring a stay button (44b) to hold the locking nail (**43**) in position.

6. Ancillary femoral instrumentation as described in claim 3 or 4 and also featuring a locking nail (43) pierced axially to accept a cross pin (45).

7. Ancillary femoral instrumentation as described in claim 1 and also featuring a mount (50) with posterior and intermediate guides including:
- end plane face (51) positioned against the surface of the distal section of the condylar bones
- interstitial tab (52) configured between the base of the mount and the end plane face
- two resection guide slots (56, 57) opening onto the plane face from the opposite side, the first slot (56) tilted toward the tab from the opposite side toward the plane face, the second (57) running more or less parallel to the tab and positioned between the tab and tilted slot.

8. Ancillary femoral instrumentation as described in claim 7 and also featuring a 4° tilt in the second slot (57) toward the tab (52).

9. Ancillary femoral instrumentation as described in claim 1, 7, or 8 and also featuring bore holes (53) on the guide part opposite the tab (52) and at various angles to accept any number of transfixation bone pins (54).
